(19)

**Europäisches Patentamt**

**European Patent Office**

**Office européen des brevets**

(11) **EP 4 417 117 A1**

(12) **EUROPEAN PATENT APPLICATION**

(43) Date of publication:
**21.08.2024 Bulletin 2024/34**

(21) Application number: **23382143.8**

(22) Date of filing: **15.02.2023**

(51) International Patent Classification (IPC):
**A61B 5/029** (2006.01)     **A61B 5/00** (2006.01)
**A61B 5/021** (2006.01)     **A61B 5/08** (2006.01)
**A61M 16/00** (2006.01)

(52) Cooperative Patent Classification (CPC):
**A61B 5/029; A61B 5/02108; A61B 5/0816;
A61B 5/7203; A61B 5/7285; A61M 16/00;**
A61B 5/0205

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Fundacion Instituto De Investigacion
Sanitaria Fundacion Jimenez Diaz
28040 Madrid (ES)**
• **Consorcio Centro de Investigación Biomédica en
Red
M.P.
28029 Madrid (ES)**

• **Asociación Centro de Investigación en
Biomateriales - CIC biomaGune
20014 San Sebastián (ES)**

(72) Inventors:
• **SANTOS OVIEDO, Arnoldo de Jesús
28040 Madrid (ES)**
• **RUIZ-CABELLO OSUNA, Jesús María
20014 San Sebastián (ES)**

(74) Representative: **Hoffmann Eitle
Hoffmann Eitle S.L.U.
Paseo de la Castellana 140, 3a planta
Edificio LIMA
28046 Madrid (ES)**

(54) **METHOD FOR ESTIMATING THE VENTRICULAR STROKE VOLUME FROM THE PULMONARY ARTERY PRESSURE**

(57)     A computer-implemented method for estimating right ventricular stroke volume from the pulmonary artery pressure, comprising: a) receiving data indicative of two or more corrected pulmonary artery pressure measurements. The two or more corrected pulmonary artery pressure measurements are one or more pulmonary artery pressure measurements over one or more cardiac cycles at one or more points in the respiratory cycle, each point corrected for the external airway pressure exerted on the heart during the cardiac cycle at the respective point in the respiratory cycle, the correction comprising a subtraction of a respiratory correction function for all of the pulmonary artery pressure measurements; and b) estimating the right ventricular stroke volume for each of the one or more cardiac cycles based on a parameter of the one or more corrected pulmonary artery pressure measurements using a predetermined relationship between the parameter and right ventricular stroke volume.

Fig. 6

EP 4 417 117 A1

**Description**

**Technical field of the invention**

The present invention pertains to the field of cardiology, particularly to a method for estimating ventricular stroke volume from the pulmonary artery pressure, a method for estimating stroke volume variation over the respiratory cycle from the pulmonary artery pressure, an apparatus of the same and a computer program product of the same.

**Background of the invention**

Estimation of left ventricle stroke volume (SV) by systemic arterial pressure waveform analysis (PWA) has been extensively studied and has multiple clinical applications, mostly in critical care. The role of right ventricle (RV) performance during acute conditions is being increasingly remarked during the last years, so there is an increasing need of pulmonary hemodynamic monitoring in clinical practice. For example, the development of acute respiratory distress syndrome (ARDS) in a patient results in a significant worsening of respiratory mechanics and gas exchange and on pulmonary hemodynamics.

In this regard, beat by beat evaluation of RV SV can bring physiological insights useful to improve clinical pulmonary hemodynamic monitoring tools. For example, RV fluid responsiveness could be predicted as well as the impact of mechanical ventilation on RV performance. As continuous beat-by-beat RV SV measurement is not possible in clinical settings with the present monitoring tools (such as thermodilution), RV SV estimation from PA PWA would be the desired alternative for such continuous beat-by-beat RV SV measurement. However, estimation of RV SV by PA PWA is affected by the effect of breathing on PA pressure measurement.

Pressure measurement of cardiovascular structures located into the thorax is affected by airway pressure which is transmitted to the pleural space. This is especially relevant for mechanically ventilated patients. Depending on the clinical application of the cardiovascular pressure measurement, some hemodynamic variables are corrected by subtracting pleural pressure from the measured intravascular pressure to obtain the transmural pressure. For example, for using the central venous pressure as a measurement of the cardiac preload, transmural central venous pressure (measured intravascular central venous pressure minus the pleural pressure) is a more accurate estimation than the intravascular pressure. In other circumstances, pressure measurement during expiration is recommended, this is the case for estimating the pulmonary circulation outflow pressure i.e. the wedge PA pressure. However, the effect of continuous breathing on PA pressure waveforms still remains to be resolved as if transmitted pressure modifies the PA pressure morphology at the beat level, the measurement of variables during each beat can lead to erroneous conclusions. This is particularly relevant for the estimation of RV SV and its change along the respiratory cycle.

Moreover, as the current SV is estimated on an average, the current monitoring tools don't allow the exploitation of all the known RV SV potential, such as the RV SV variation (RV SVV) during the respiration cycle. SVV is a phenomenon occurring under mechanical ventilation in which the arterial pulse pressure falls during inspiration and rises during expiration due to changes in intra-thoracic pressure secondary to positive pressure. The SVV is known to have a very high sensitivity and specificity when compared to traditional indicators of volume status and their ability to determine fluid responsiveness when evaluating systemic blood circulation. However, currently the RV SV is not assessed in a continuous beat-by-beat manner, so it cannot be used to estimate the SVV and to predict the response to right ventricular fluid administration.

There is therefore a need for a continuous beat-by-beat RV SV estimation from the PA PWA with high confidence avoiding the effect of continuous breathing, and for a way to predict the response to right ventricular fluid administration.

**Summary of the invention**

A first aspect of the invention refers to a computer-implemented method for estimating ventricular stroke volume from the pulmonary artery pressure. The method comprises:

a) receiving data indicative of two or more corrected pulmonary artery pressure measurements. The two or more corrected pulmonary artery pressure measurements are one or more pulmonary artery pressure measurements over one or more cardiac cycles at one or more points in the respiratory cycle. Each point is corrected for the external airway pressure exerted on the heart during the cardiac cycle at the respective point in the respiratory cycle, the correction comprising a subtraction of a respiratory correction function for all of the pulmonary artery pressure measurements; and

b) estimating the ventricular stroke volume for each of the one or more cardiac cycles based on a parameter of the one or more corrected pulmonary artery pressure measurements using a predetermined relationship between the parameter and ventricular stroke volume.

In a preferred embodiment of the first aspect of the invention, the respiratory correction function is computed through a method. The method comprises:

a) receiving data indicative of two or more pulmonary artery pressure cycles;
b) receiving data indicative of two or more respiratory cycles wherein the respiratory data is synchronized with the pulmonary artery pressure data;
c) detecting a predetermined position of the at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles of step a), and rearranging each pulmonary artery pressure cycle of step b) according to their position in time, with respect to the aforesaid predetermined position of these respiratory cycles;
d) defining a discrete signal by plotting each of a determined measurement of the pulmonary artery pressure cycles in relation to the time of its respective respiratory cycle's starting point obtained as described in step c) above, as an ordinate within the respiratory cycle; and
e) computing the respiratory correction function by reconstructing a continuous signal from the defined discrete signal.

In a more preferred embodiment of the first aspect of the invention, the reconstruction of the continuous signal from the discrete signal is done through interpolation. More preferably, the interpolated signal is performed through a smoothing splines algorithm.

In another more preferred embodiment of the first aspect of the invention, the predetermined position of the respiratory cycle is the starting position of the respiratory cycle.

In another more preferred embodiment of the first aspect of the invention, the correction comprising a subtraction comprises subtracting to each of the pulmonary artery pressure measurements the value of each corresponding measurement of the respiratory correction function. The respiratory correction function is synchronised with the pulmonary artery pressure measurements according to their respective point in the respiratory cycle and the respiratory correction function is extended in a repeated pattern at intervals defined by respiratory cycle.

In another more preferred embodiment of the first aspect of the invention, the determined measurement is the final diastolic pressure value of each pulmonary artery pressure cycle.

In another more preferred embodiment of the first aspect of the invention, the parameter is the difference between the systolic and diastolic corrected pressure in one cardiac cycle. Alternatively, the parameter is the area under the curve of the systolic portion of the corrected pressure in one cardiac cycle. In another alternative, the parameter is the standard deviation of the corrected pressure over one cardiac cycle.

In another more preferred embodiment of the first aspect of the invention, respiratory correction function is scaled such that its minimum value is 0.

In another more preferred embodiment of the first aspect of the invention, the predetermined relationship is a linear relationship computed through a calibration function between the ventricular stroke volume and the parameter of the pulmonary artery pressure. Even more preferably, the calibration function is the quotient between the scaled thermodilution cardiac output divided by heart rate and the parameter of the of the pulmonary artery pressure.

A second aspect of the invention refers to a computer-implemented method for estimating stroke volume variation (SVV) over the respiratory cycle from the pulmonary artery pressure. The method comprises:

a) estimating ventricular stroke volume (SV) according to any of the computer-implemented methods of the first aspect of the invention,
b) estimating SVV over the respiratory cycle for each respiratory cycle as:

$$SVV = \frac{SV_{max} - SV_{min}}{SV_{mean}}$$

wherein SVmax is the maximum SV during such cycle, SVmin is the minimum SV during such cycle, and SVmean is the mean SV during such cycle.

A third aspect of the invention refers to an apparatus comprising a processor and a memory unit. The memory comprises instructions that when executed by the processor, estimates the ventricular stroke volume according to any of the computer-implemented methods of the first aspect of the invention.

In a preferred embodiment of the third aspect of the invention, the memory unit further comprises instructions that, when executed by the processor, estimates stroke volume variation (SVV) over the respiratory cycle from the pulmonary artery pressure according to the computer-implemented method of the second aspect of the invention.

A fourth aspect of the invention refers to a computer program product configured for estimating a ventricular stroke volume from a ventricle arterial pressure according to any of the computer-implemented methods of the first aspect of the invention.

In a preferred embodiment of the fourth aspect of the invention, the computer program product is further configured to estimate stroke volume variation (SVV) over the respiratory cycle from the pulmonary artery pressure according to the second aspect of the invention.

## Brief description of the drawings

To enable better understanding of the present disclosure, and to show how the same may be carried into effect, reference will now be made, by way of example only, to the accompanying schematic drawings, in which:

Fig. 1A shows an example of a scatter plot between SV estimated from the non-corrected pulmonary artery pressure signal area and a reference SV according to one or more embodiments.

Fig. 1B shows an example of a scatter plot between SV estimated from the non-corrected but shifted pulmonary artery pressure signal area and a reference SV according to one or more embodiments.

Fig. 1C shows an example of a scatter plot between SV estimated from the corrected pulmonary artery pressure signal area and a reference SV according to one or more embodiments.

Fig. 2A shows an example of a non-corrected pulmonary artery pressure amplitude spectrum according to one or more embodiments.

Fig. 2B shows an example of the corrected pulmonary artery pressure amplitude spectrum of the signal of 2A, according to one or more embodiments.

Fig. 3A shows a comparison of the effect of the correction on both healthy subjects (baseline) and subjects with ARDS for the respiratory component of the amplitude spectrum according to one or more embodiments.

Fig. 3B shows a comparison of the effect of the correction on both healthy subjects (baseline) and subjects with ARDS for the cardiac component of the amplitude spectrum according to one or more embodiments.

Fig. 4A shows an example of a non-corrected pulmonary artery pressure signal (upper graph) and an example of the corresponding respiratory cycles signal (lower graph) according to one or more embodiments.

Fig. 4B shows the SV beat-by-beat values of the non-corrected pulmonary artery pressure signal (upper graph) and the corresponding respiratory cycles signal (lower graph) of Fig. 3A according to one or more embodiments.

Fig. 4C shows an example of a corrected pulmonary artery pressure signal (upper graph) and an example of the corresponding respiratory cycles signal (lower graph) according to one or more embodiments.

Fig. 4D shows the SV beat-by-beat values of the corrected pulmonary artery pressure signal (upper graph) and the corresponding respiratory cycles signal (lower graph) of Fig. 3A according to one or more embodiments.

Fig. 5A shows an example of a non-corrected pulmonary artery pressure signal according to one or more embodiments.

Fig. 5B shows an example of a respiratory signal according to one or more embodiments.

Fig. 5C shows an example of a respiratory correction function signal for the non-corrected pulmonary artery pressure signal of Fig. 1A according to one or more embodiments.

Fig. 6 shows a flow diagram of a computer-implemented method for estimating ventricular stroke volume from the pulmonary artery pressure according to one or more embodiments.

Fig. 7A shows an example of a non-corrected pulmonary artery pressure signal according to one or more embodiments.

Fig. 7B shows an example of the respiratory correction function signal of Fig. 5C extended in a repeated pattern at intervals defined by respiratory cycle according to one or more embodiments.

Fig. 7C shows the corrected pulmonary artery pressure signal of Fig. 1A corrected with the respiratory correction function signal of Fig. 1B according to one or more embodiments.

Fig. 8 shows a comparison table between the correlation of the pulse pressure, standard deviation and area under the curve parameters of the corrected and non-corrected PAP signals and the ventricular stroke volume.

Fig. 9 shows a flow diagram of a computer-implemented method for estimating ventricular stroke volume variation over the respiratory cycle from the pulmonary artery pressure according to one or more embodiments.

Fig. 10A shows a correlation plot between a reference SVV and the SVV calculated from the non-corrected values of the pulmonary artery pressure according to one or more embodiments.

Fig. 10B shows a correlation plot between a reference SVV and the SVV calculated from the corrected values of the pulmonary artery pressure according to one or more embodiments.

Fig. 10C shows a correlation plot between a reference SVV and the SVV calculated from the non-corrected standard deviation of the pulmonary artery pressure according to one or more embodiments.

Fig. 10D shows a correlation plot between a reference SVV and the SVV calculated from the corrected standard deviation of the pulmonary artery pressure according to one or more embodiments.

Fig. 10E shows a correlation plot between a reference SVV and the SVV calculated from the non-corrected area under the curve of the pulmonary artery pressure according to one or more embodiments.

Fig. 10F shows a correlation plot between a reference SVV and the SVV calculated from the corrected area under the curve of the pulmonary artery pressure according to one or more embodiments.

Fig. 11 shows an example of a stroke volume signal over the respiration cycle according to one or more embodiments.

Fig. 12 shows a scheme of an apparatus according to one or more embodiments.

## Description of the invention

### Description

The methodology presented herein estimates, among other things, the right ventricle stroke volume (RV SV) from the pulmonary artery pressure waveform analysis (PA PWA) avoiding the effect of continuous breathing in a continuous beat-by-beat manner.

In this sense in the present invention, and by using the methodology described herein, it has been demonstrated the feasibility of continuously estimating the RV SV with high confidence, which opens the door for new pulmonary hemodynamic monitoring solutions, particularly focused in the right side of the heart. Several clinical applications are opened as herein described.

The method of the present invention relies on the fact that if pulmonary hemodynamic signals, such as pulmonary pressure signals, are reordered according to the respiratory cycle time at which the heart-beat occurs, a clear respiratory modulation can be observed. Therefore, the PA pressure signals can be effectively cleaned from these respiratory modulation, generating corrected PAP signals. The corrected PA pressure signals are therefore cleaned from the effect of continuous breathing and are thus feasible for RV SV estimation. It has been demonstrated that the correction herein provided eliminates the respiratory components of the PAP pressure signal without affecting the cardiac components of such, leading to a PA pressure signal which can be easily correlated to the RV SV. Moreover, this allows for right ventricle stroke volume variability (SVV) high quality estimation in a beat-by-beat and continuous basis, which opens new clinical applications for the PA catheter solutions.

It is noted that from herein after, reference will be made to SV and RV SV indistinctively, but the stroke volume is referred preferably to the right ventricle in all cases.

It is thus an object of the present invention to provide a new method for estimating the ventricular stroke volume from the pulmonary artery pressure which does not have the disadvantages of the methods used in the prior art as discussed above. Therefore, one aspect of the invention refers to a method (from hereinafter method of the invention) for the estimating ventricular stroke volume from the pulmonary artery pressure, which comprises:

> a) receiving data indicative of two or more corrected pulmonary artery pressure measurements, wherein the two or more corrected pulmonary artery pressure measurements are one or more pulmonary artery pressure measurements over one or more cardiac cycles at one or more points in the respiratory cycle, each point corrected for the external airway pressure exerted on the heart during the cardiac cycle at the respective point in the respiratory cycle, the correction comprising a subtraction of a respiratory correction function for all of the pulmonary artery pressure measurements; and

> b) estimating the ventricular stroke volume for each of the one or more cardiac cycles based on a parameter of the one or more corrected pulmonary artery pressure measurements using a predetermined relationship between the parameter and ventricular stroke volume.

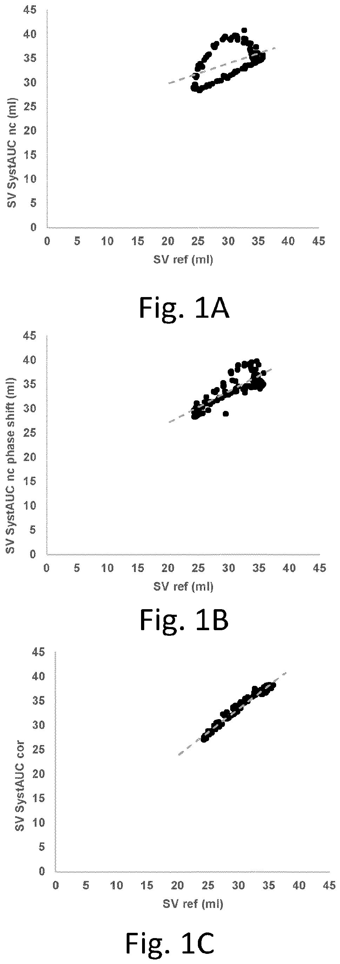

As indicated in Fig. 1A, a predetermined relationship can be stablished between the pulmonary artery pressure (PAP) measurements and the ventricular stroke volume (SV) however this relationship function is surjective as each PAP value can be associated to more than one SV value. When the phase shift of the PAP measurements is corrected as illustrated in Fig. 1B, it can be noticed that the relationship starts to become more bijective, although it cannot be used with confidence. However, when the PAP measurements are corrected to eliminate the effect of respiration on the signal through a subtraction of a respiratory correction function, it can be seen in Fig. 1C that the predetermined relationship becomes bijective, effectively providing a functional way of estimating the ventricular SV value. It is noted that the term subtraction in the present application is preferably understood as subtracting the values, independently on the sign of the values of any of the signals, such that if a measurement of the second term (i.e. the subtrahend) of the subtraction (e.g. the respiratory correction function) is negative, the negative value is subtracted from the measurement value of the first term (i.e. the minuend) of the subtraction (e.g. the pulmonary artery pressure signal). Therefore the result in such case is equivalent to adding the absolute value of the subtrahend to the minuend.

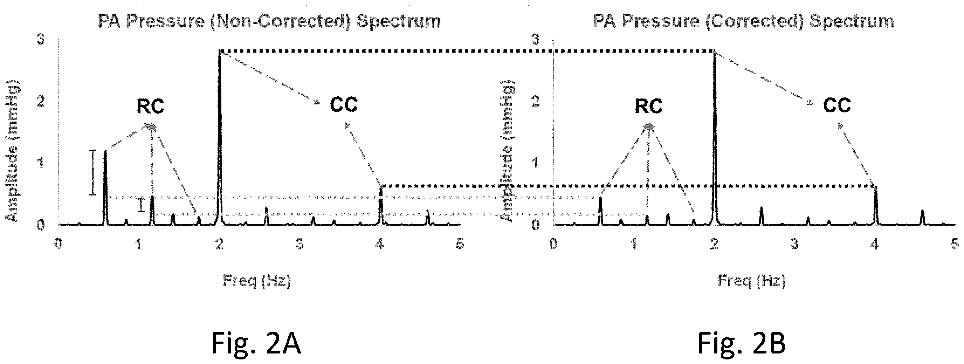

As illustrated in Fig. 2A-B, one of the main advantages of the method of the present invention, is that it significantly reduces the respiratory components (RC) of PAP signal without affecting the cardiac components (CC) of the PAP signal. Fig. 2A shows an example of a pulmonary artery flow amplitude spectrum according to one or more embodiments. IN Fig. 2A it can be seen that a non-corrected signal comprises both RC and CC and in Fig 2B it can be seen that while the RC components amplitude is reduced, the CC components amplitude is not affected.

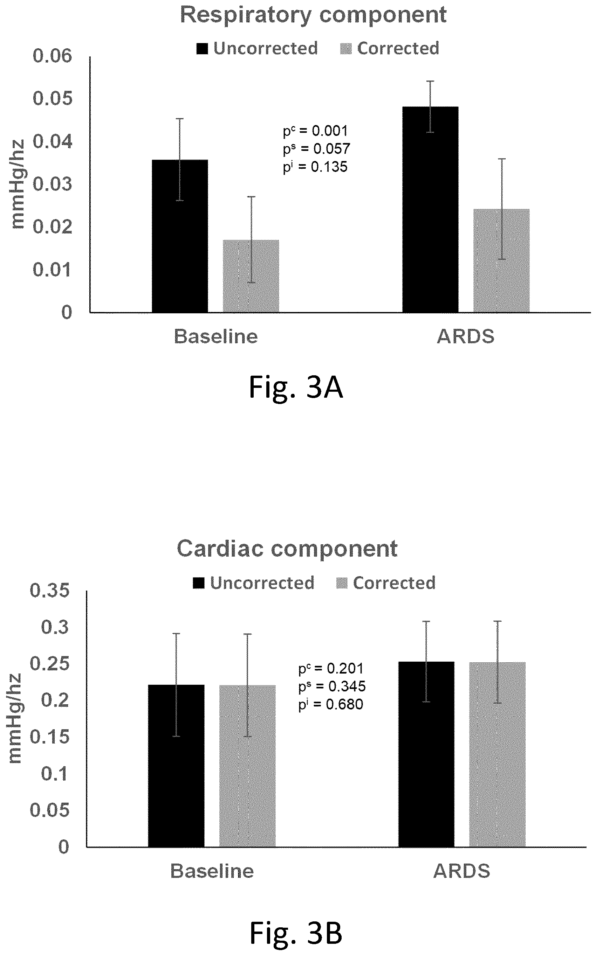

Advantageously, this method works independently on the state of the subject. This is further illustrated in Fig 3A-B, wherein it is shown how in Fig. 3A, the respiratory components of both the healthy subject (baseline) and after acquiring ARDS, are reduced when the correction is applied, while in Fig. 3B, the cardiac component is not affected by the correction in either case.

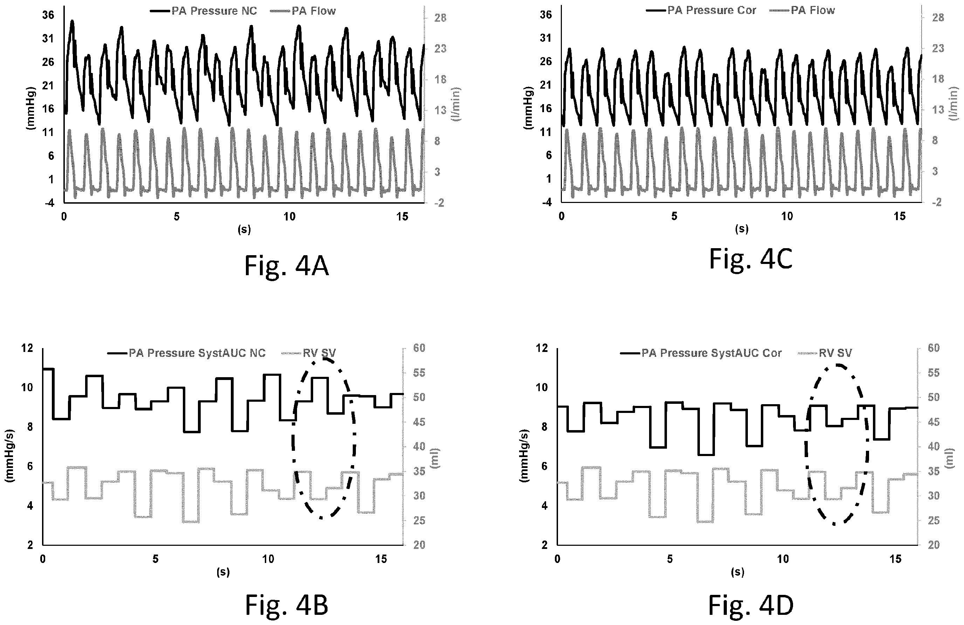

As a further illustrative example of the present methodology, please note that Figs. 4A-D show the effect on the correlation between the PAP and the SV achieved by the proposed correction. Fig. 4A shows measurements from both a non-corrected PAP signal (upper graph) and the ventricular SV signal (lower graph). Fig. 4B shows the beat-by-beat values of each signal, which show that the correlation between both signals is poor. On the other hand, Fig. 4C shows measurements from both the corrected version of the non-corrected PAP signal of Fig. 4A (upper graph) and the ventricular SV signal (lower graph). Fig. 4C shows the beat-by-beat values of each signal of Fig. 4C, which show that the correlation between both signals is now very high.

Therefore, in a preferred embodiment of the first aspect of the invention, the respiratory correction function is computed through a method, which comprises:

> a) receiving data indicative of two or more pulmonary artery pressure cycles;
> b) receiving data indicative of two or more respiratory cycles wherein the respiratory data is synchronized with the pulmonary artery pressure data;
> c) detecting a predetermined position of the at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles of step a), and rearranging each pulmonary artery pressure cycle of step b) according to their position in time, with respect to the aforesaid predetermined position of these respiratory cycles;
> d) defining a discrete signal by plotting each of a determined measurement of the pulmonary artery pressure cycles in relation to the time of its respective respiratory cycle's starting point obtained as described in step c) above, as an ordinate within the respiratory cycle; and
> e) computing the respiratory correction function by reconstructing a continuous signal from the defined discrete signal.

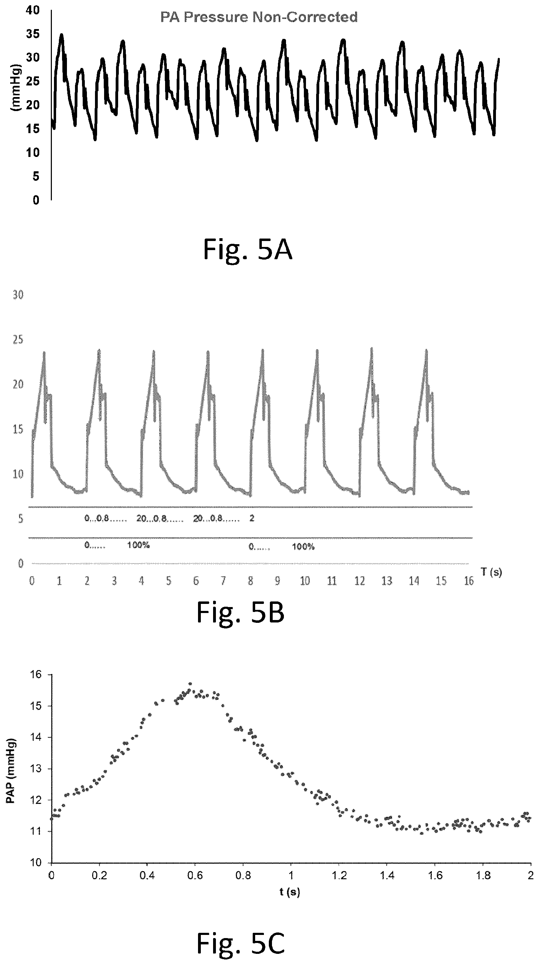

As indicated in Fig. 5A, it is noted that the received data of step a) indicative of two or more non-corrected pulmonary artery pressure cycle measurements is affected by the respiratory cycle. This can be noticed in the amplitude variability of the cyclic signal, which should remain within the same minimum and maximum amplitude values.

As shown with respect to Fig. 5B, the received data of step b) of two or more respiratory cycles is synchronized in time with the pulmonary artery pressure data. Moreover, as referred in step c), a predetermined position of the at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles of step a) can be detected. In the particular example of Fig. 5B, the respiratory cycle has a 2 second duration, the predetermined position comprised within the 0% of the cycle and the 100% of the cycle. Then, each pulmonary artery pressure cycle of step b) is rearranged according to their position in time, with respect to the aforesaid predetermined position of these respiratory cycles.

To practice the method of the present invention according to at least this preferred embodiment, it is deemed necessary to detect with high precision the determined position of the respiratory such as the starting point of each cycle; and as indicated for example in Fig. 5B, once the start of the respiratory cycle is located, a new definition of the relative times of the respiratory cycle is made with respect to the same cycle. That is to say that the temporalis events begin to be considered relative to the respiratory cycle which begins to be considered as 0 once each respirable cycle begins. Taking into account that each point of the respiratory cycle can be considered in units of time whose maximum will be the duration of the respiratory cycle or in percentages or fractions with respect to each cycle, being the beginning 0 and the maximum 100% or 1. This is considered as such because the pulmonary artery pressure cycles will be reordered based on this relative position as indicated in step c) above. For the reordering of the cardiac cycles it is important to establish the position of the measurements of each pulmonary artery pressure cycle relative to the respiratory cycle so that the beginning of each pulmonary artery pressure cycle is placed and reordered relative to its position to the respiratory cycle. This is further illustrated in Fig. 5c) wherein the numbering of the sampling points (corresponding to the hemodynamic or cardiac cycle) is carried out according to the beginning of each respiratory cycle.

As illustrated in Fig. 5C, the discrete signal of step d) can be determined, by plotting each of the determined measurement of the pulmonary artery pressure cycles in relation to the time of the predetermined point of its respective respiratory cycle as described in step c) above, as an ordinate within the respiratory cycle. As the respiratory cycle duration may vary between cycles, the respiratory cycle of reference for plotting each determined measurement is scaled according to its proportional duration, using the above mentioned 0-100% scale defined for the respiration cycle.

Finally, the respiratory correction function can be obtained by reconstructing a continuous signal from the defined discrete signal. It is noted that the respiratory correction function may be reconstructed in several ways as described further below.

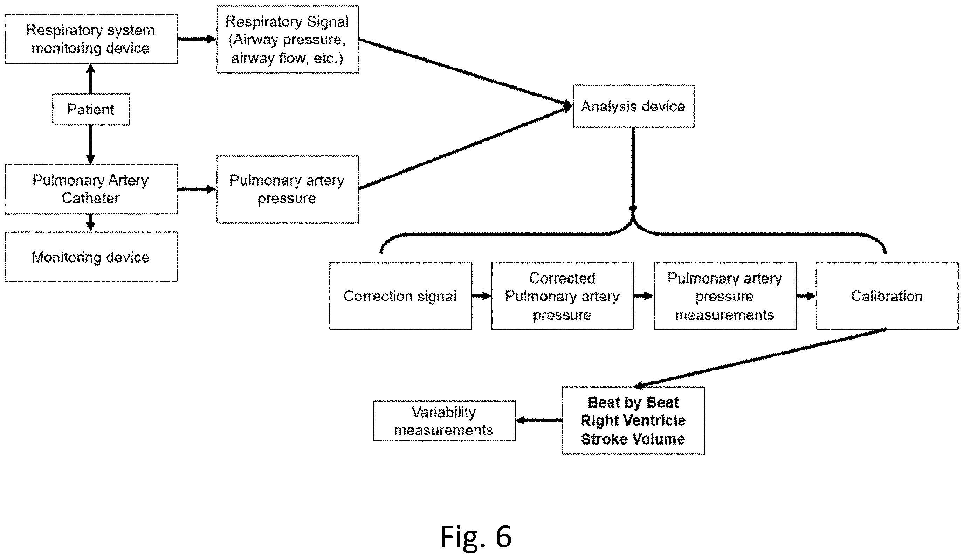

As reflected above and as indicated in Fig. 6, it is noted that, the method of the invention requires two signals: a respiratory and a cardiac or hemodynamic signal, for obtaining the respiration cycle and the cardiac cycle of the subject. It is noted that this respiratory signal may result, but no limited to: changes in the chest volume, changes in the airway pressure, changes in the air flow or even from the cardiac or hemodynamic signal itself. It is also noted that the respiratory cycle measurements are obtained from a respiratory system monitoring device connected to the patient such as a capnometry, a respiratory flow, an airway pressure, transthoracic electrical impedance, a plethysmogram, or a respiratory temperature. It is also noted that the pulmonary artery pressure cycle measurements may be obtained through a pulmonary artery catheter connected to the patient.

Therefore, in a more preferred embodiment of the first aspect of the invention, the reconstruction of the continuous signal from the discrete signal is done through interpolation. The skilled person will note that there are several ways of performing interpolation to reconstruct the signal such as piecewise constant interpolation, linear interpolation, polynomial interpolation, spline interpolation or mimetic interpolation. More preferably, the interpolated signal is performed through a spline algorithm, even more preferably through a smoothing spline algorithm.

In another more preferred embodiment of the first aspect of the invention, the predetermined position of the respiratory cycle is the starting position of the respiratory cycle. It is noted that the respiratory cycle is agreed to start with the inspiration and end with the exhalation. Therefore, the starting position of the respiratory cycle, is more preferably the start of the inspiration phase. However, it is noted that the skilled person may define the respiratory cycle in alternative ways such as starting with the expiration, and thus in some other embodiments the predetermined position may not be the starting position of the respiratory cycle, and that the skilled person may use other position of the respiratory cycle as the predetermined position.

In another preferred embodiment of the first aspect of the invention, the correction comprising a subtraction comprises subtracting to each of the pulmonary artery pressure measurements the value of each corresponding measurement of the respiratory correction function, wherein the respiratory correction function is synchronised with the pulmonary artery pressure measurements according to their respective point in the respiratory cycle, and wherein the respiratory correction function is extended in a repeated pattern at intervals defined by respiratory cycle.

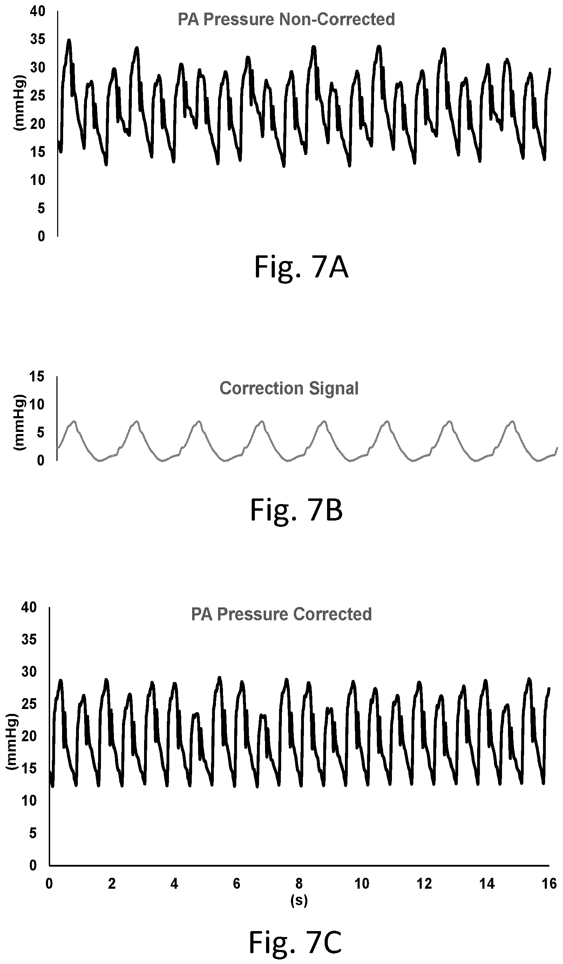

As shown with respect to Figs. 7A-C, the subtraction is performed by subtracting to each of the pulmonary artery pressure measurements shown in Fig. 7A, the value of the corresponding the value of each corresponding measurement of the respiratory correction function shown in Fig. 7B, which effectively results in the corrected pulmonary artery pressure measurements of Fig. 7C. The pulmonary artery pressure measurements shown in Fig. 7A and the respiratory correction function shown in Fig. 7B are synchronised according to their respective point in the respiratory cycle.

It is noted that the respiratory correction function has a period of just one respiratory cycle, so both respiratory correction function and the pulmonary artery pressure measurements are synchronised according to the respiratory cycle. Moreover, when the pulmonary artery pressure measurements signal is longer than the respiratory cycle, the respiratory correction function is extended in a repeated pattern at intervals defined by respiratory cycle. This can be seen for example in Fig. 7B, wherein as the pulmonary artery pressure signal of Fig. 7A is longer than the respiratory cycle, the respiratory correction function of Fig. 5C has been extended in a repeated pattern at intervals defined by respiratory cycle to achieve the respiratory correction function of Fig. 7C.

In another more preferred embodiment of the first aspect of the invention, the determined measurement is the final diastolic pressure value of each pulmonary artery pressure cycle. Advantageously, this provides a determined measurement that is highly affected by the transmission of the respiratory pressure and thus is significantly representative of the transmitted pressure.. The final diastolic pressure value can be measured in several ways as know by the person skilled in the art. For example, the final diastolic pressure value can be measured by computing the local minimum of the PAP-time curve between the final diastolic measurements and the initial systolic measurements, or by computing the local maxima of the second derivative of the PAP-time curve of the final diastolic measurements and the initial systolic measurements, or by computing the first zero-crossing point of the third derivative of the PAP-time curve of the final diastolic measurements and the initial systolic measurements, or by computing the intersection point of two tangents, a first tangent traced on the final diastolic measurements and the other one traced on the initial systolic measurements.

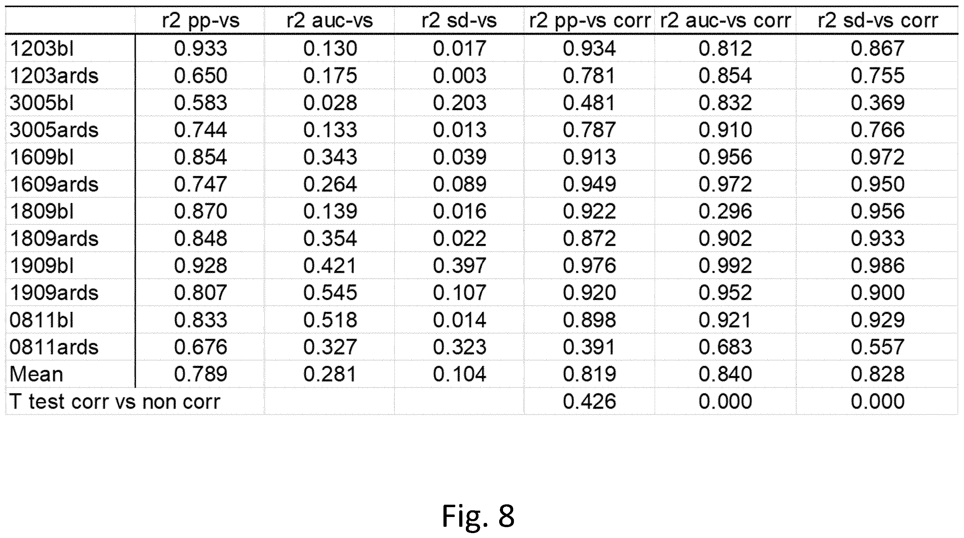

In another preferred embodiment of the first aspect of the invention, the parameter is the difference between the systolic and diastolic corrected pressure in one cardiac cycle. As shown in the example, with respect to tables 2a and 2b and with respect to Fig. 8, the difference between the systolic and diastolic corrected pressure in one cardiac cycle, i.e. the pulse pressure is a parameter of the corrected PAP signal that provides an appropriate estimate for the right ventricular stroke value. More preferably, the method comprises receiving data indicative of more than two corrected pulmonary artery pressure measurements, preferably three, more preferably four, more preferably five, more preferably 6, 7, 8, 9, 10, 11, 12, 13, 14, 15, 16, 17, 18, 19 or at least preferably 20, corrected pulmonary artery pressure measurements.

In another preferred embodiment of the first aspect of the invention, the parameter is the area under the curve of the systolic portion of the corrected pressure in one cardiac cycle. As shown in the example, with respect to tables 2a and 2b and with respect to Fig. 8, the area under the curve of the systolic portion of the corrected pressure in one cardiac cycle is a parameter of the corrected PAP signal that provides an appropriate estimate for the right ventricular stroke value. It is noted that more than two measurements may be required in other to compute the area under the curve of the systolic portion of the corrected pressure in one cardiac cycle. Therefore, more preferably, the method comprises receiving data indicative of more than two corrected pulmonary artery pressure measurements, preferably three, more preferably four, more preferably five, more preferably 6, 7, 8, 9, 10, 11, 12, 13, 14, 15, 16, 17, 18, 19 or at least preferably 20, corrected pulmonary artery pressure measurements.

In another preferred embodiment of the first aspect of the invention, the parameter is the standard deviation of the corrected pressure over one cardiac cycle. As shown in the example, with respect to tables 2a and 2b and with respect to Fig. 8, the standard deviation of the corrected pressure over one cardiac cycle.is a parameter of the corrected PAP signal that provides an appropriate estimate for the right ventricular stroke value. It is noted that more than two measurements may be required in other to compute the standard deviation of the corrected pressure over one cardiac cycle. Therefore, more preferably, the method comprises receiving data indicative of more than two corrected pulmonary artery pressure measurements, preferably three, more preferably four, more preferably five, more preferably 6, 7, 8, 9, 10, 11, 12, 13, 14, 15, 16, 17, 18, 19 or at least preferably 20, corrected pulmonary artery pressure measurements.

In another preferred embodiment of the first aspect of the invention, the respiratory correction function is scaled such that its minimum value is 0. Advantageously, this allows for the pulmonary artery pressure to be corrected such that the pressure values are not displaced towards lower of higher numbers than those of a PAP signal. This further allows for using the same predetermined relationship between the PAP and the ventricular SV as known in the art to obtain the estimated SV.

In another preferred embodiment of the first aspect of the invention, the predetermined relationship is a linear relationship computed through a calibration function between the ventricular stroke volume and the parameter of the pulmonary artery pressure. Fig. 6 shows a flow diagram of a method according to at least this embodiment, wherein the predetermined relationship is a linear relationship computed through a calibration function. This calibration may be applied to any of the parameters of the corrected pulmonary artery pressure. Advantageously this provides a simpler way to obtain the estimated ventricular SV values. The relationship may be used by different ways such as using a look up table or computing the output using the calibration.

In another more preferred embodiment of the first aspect of the invention, the calibration function is the quotient between the scaled thermodilution cardiac output (TDCO) divided by heart rate (HR) and the parameter of the of the pulmonary artery pressure. The quotient between the TDCO and the HR provides the measured stroke volume (SVm):

$$SV_m = \frac{TDCO}{HR}$$

and the calibration function (CF) can be computed as the quotient between the SVm and the parameter of the of the pulmonary artery pressure, either the pulse pressure (PP), the AUC or the SD of the PP.

$$CF = \frac{SV_m}{PP}; \quad CF = \frac{SV_m}{AUC_{PP}}; \quad or \quad CF = \frac{SV_m}{SD_{PP}}$$

It is noted that the skilled person would know other forms of calibrating the relationship between the SV and the PP, or it may not be even calibrated at all.

From the estimated right ventricle SV, it is feasible to calculate the right ventricle SV variation (SVV) over the respiratory cycle which is known to have a very high sensitivity and specificity when compared to traditional indicators of volume status and their ability to determine fluid responsiveness. However, the estimated SVV proposed herein is computed in a continuous way, so the applications of the SVV parameter can be expanded.

Therefore a second aspect of the invention refers to a computer-implemented method for estimating stroke volume variation (SVV) over the respiratory cycle from the pulmonary artery pressure, which comprises:

a) estimating ventricular stroke volume (SV) according to any of the computer-implemented methods of the first aspect of the invention,
b) estimating SVV over the respiratory cycle for each respiratory cycle as:

$$SVV = \frac{SV_{max} - SV_{min}}{SV_{mean}}$$

wherein SVmax is the maximum SV during such cycle, SVmin is the minimum SV during such cycle, and SVmean is the mean SV during such cycle.

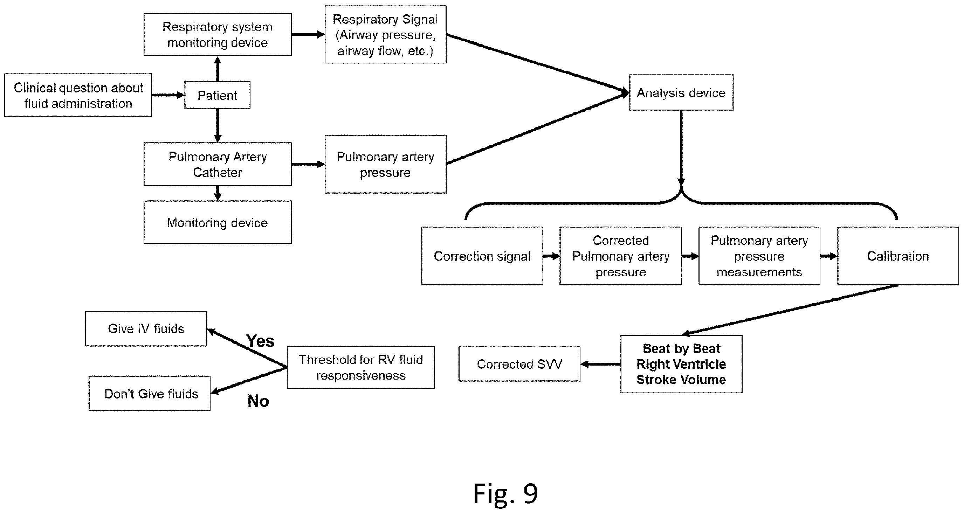

As shown with respect to Fig. 9 according to one or more embodiments, the beat-by-beat right ventricle SV computed from any of the computer-implemented methods of the first aspect of the invention, can be used to obtain an estimated SVV, that is also corrected such that is not affected by the transmission of pressure during the respiration cycle. It is noted that although Fig. 9 shows a particular flow diagram, the SV may be estimated according to any of the embodiments of the first aspect of the invention.

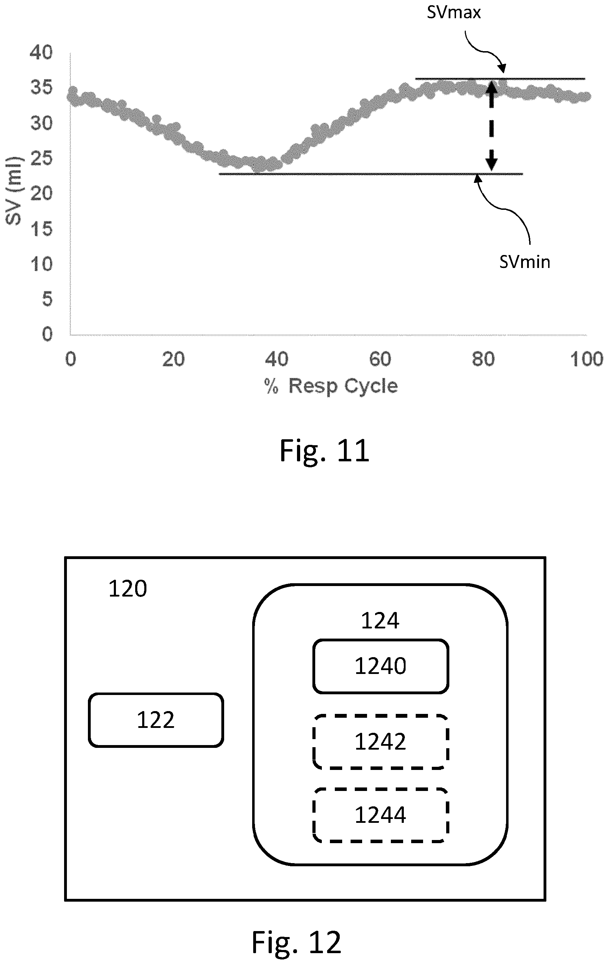

As an illustrative example of the SVV calculation, note that Fig. 11 shows an estimated SV over the respiratory cycle, wherein the SVmax and SVmin are noted. The SVmean may be computed in several ways as known in the art. For example. The mean between the SVmax and SVmin may be computed. Alternatively, the mean of all the SV measurements may be used. If the mean between the SVmax and SVmin is computed, the SVV may be then estimated as:

$$SVV = \frac{SV_{max} - SV_{min}}{\left(\dfrac{SV_{max} + SV_{min}}{2}\right)}$$

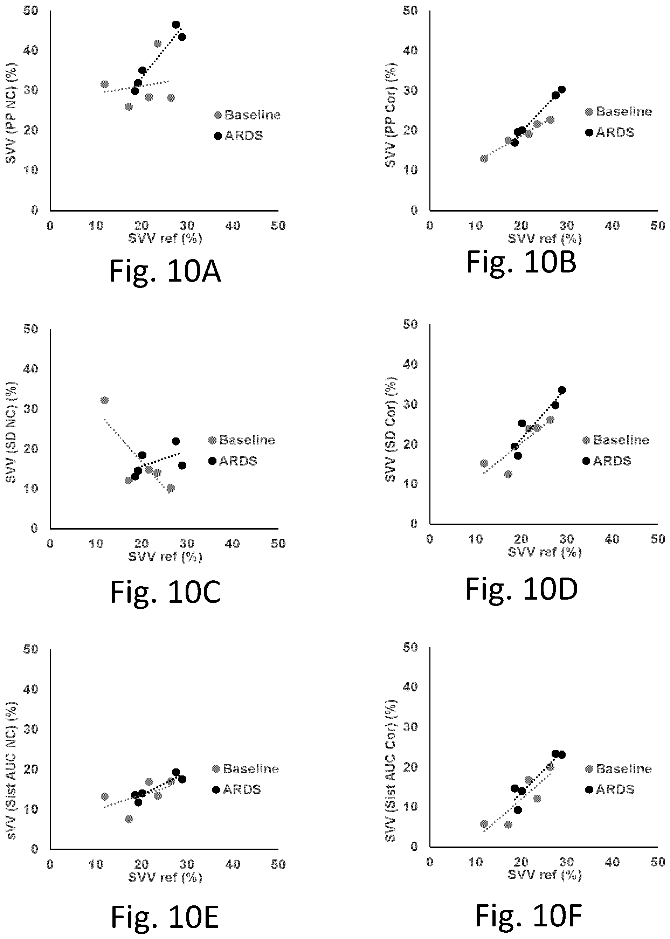

As shown with respect to Figs 10.A-F and table 3, any of the parameters for the corrected PAP are provide an appropriate estimate for the right ventricular stroke value. In Figs. 10A and 10B, it can be seen hat the difference between the systolic and diastolic corrected pressure in one cardiac cycle, i.e. the pulse pressure is a parameter of the corrected PAP signal that provides an appropriate estimate for the right ventricular stroke value as it has a similar value as the reference RV SV, while this is not the case for the uncorrected PAP signal. Similarly, in Figs. 10C and 10D, it can be seen that the standard deviation of the corrected pressure over one cardiac cycle is a parameter of the corrected PAP signal that provides an appropriate estimate for the right ventricular stroke value as it has a similar value as the reference RV SV, while this is not the case for the uncorrected PAP signal. Also, in Figs. 10E and 10F, it can be seen that the area under the curve of the systolic portion of the corrected pressure in one cardiac cycle is a parameter of the corrected PAP signal that provides an appropriate estimate for the right ventricular stroke value as it has a similar value as the reference RV SV, while this is not the case for the uncorrected PAP signal.

An aspect of the invention refers to the use of the estimated SV according to any of the embodiments of the first aspect of the invention for computing the cardiac output of the right ventricle. This can be achieved through the beat-by-beat SV estimation for a minute or the extrapolation of several beats to a minute-long period. Cardiac output estimation is a measure used for the management of different types of patients mainly when they are cardiovascular unstable or at risk of cardiovascular instability. In particular it may be used (although not limited to) in patients using a pulmonary artery catheter because of clinical interest in monitoring pulmonary vascular function or right ventricular function.

An alternative to such aspect of the invention refers to the use of the estimated SV to evaluate interventions that produce a quick systolic volume change. This may be for example passive leg elevation, change in mechanical ventilation settings, effect of fast-acting medication among others.

Another alternative to such aspect of the invention refers to the use of the estimated SVV over the respiratory cycle according to any of the embodiments of the second aspect of the invention for predicting the response to right ventricular fluid administration. As shown in Fig 9, the corrected SVV over the respiratory cycle estimated according to any of the embodiments of the second aspect of the invention, may be used to predict the response to a right ventricular fluid administration, such that a threshold may be determined for the right ventricle fluid responsiveness computed form the estimated SVV over the respiratory cycle. Therefore, according to the threshold, it may be then determined if appropriate to give intravenous fluids to the patient. It is known that a higher the SVV the more the effect of the respiration over the haemodynamics, which is indicative of a higher probability of a positive response to a right ventricular fluid administration. This may be further used to evaluate and compare the response to ventricular administration between the right and left ventricle, including the response to drug administration.

Another alternative to such aspect of the invention refers to the use of the estimated SV over the respiratory cycle to compute the mechanical ventilation impact over the right ventricular function. From the SV over the respiratory cycle, it can be assessed in which phase the systolic volume increases or decreases. This assessment could be complemented by other measurements on the corrected pulmonary artery pressure curve. This may be further used to evaluate and compare the effect of mechanical ventilation between the right and left ventricle.

A third aspect of the invention as shown in Fig. 12 refers to an apparatus 120 comprising a processor 122 and a memory unit 124, wherein the memory comprises instructions 1240 that when executed by the processor, estimates the ventricular stroke volume according to any of the computer-implemented methods of the first aspect of the invention.

It is noted that the processor may comprise one or more processing units, such as a microprocessor, GPU, CPU, multi-core processor or the like. Similarly, the memory may comprise one or more volatile or non-volatile memory devices, such as DRAM, SRAM, flash memory, read-only memory, ferroelectric RAM, hard disk drives, floppy disks, magnetic tapes, optical disks or the like.

In a preferred embodiment of the third aspect of the invention, the memory unit further comprises instructions 1244 that, when executed by the processor, computes the cardiac output of the right ventricle, or evaluates interventions that produce a quick systolic volume change from the estimated ventricular SV.

In a preferred embodiment of the third aspect of the invention, the memory unit further comprises instructions 1242 that, when executed by the processor, estimates stroke volume variation (SVV) over the respiratory cycle from the pulmonary artery pressure according to any of the computer-implemented methods of the second aspect of the invention.

In a further preferred embodiment of the third aspect of the invention, the memory unit further comprises instructions 1244 that, when executed by the processor, predicts the response to ventricular fluid administration or compute the mechanical ventilation impact over the right ventricular function from the estimated SVV.

A fourth aspect of the invention refers to a computer program product configured for estimating a ventricular stroke volume from a ventricle arterial pressure according to any of the computer-implemented methods of the first aspect of the invention.

In a preferred embodiment of the fourth aspect of the invention, the computer program product is further configured to compute the cardiac output of the right ventricle or evaluate interventions that produce a quick systolic volume change from the estimated ventricular SV. In a preferred embodiment of the fourth aspect of the invention, the computer program product is further configured to estimate stroke volume variation (SVV) over the respiratory cycle from the pulmonary artery pressure according to any of the computer-implemented methods of the second aspect of the invention.

In a further preferred embodiment of the fourth aspect of the invention, the computer program product is further configured to predict the response to ventricular fluid administration or compute the mechanical ventilation impact over the right ventricular function from the estimated SVV.

### Examples

### Material and methods

This study was conducted at the Hedenstierna laboratory, Uppsala University, Sweden and approved by the Institutional Animal Care and Use Committee. Five pigs (sus scrofa domesticus, Norwegian-Yorkshire breed) (31.8 $\pm$

6.0 kg) were studied. These animals are part of previous published studies[3,4] and were chosen if the same PEEP was applied before and after ARDS development, did not receive vasoactive drugs nor present spontaneous respiratory efforts during measurements and had simultaneous continuous recording of the airway and PA pressure and flow.

*Anaesthesia and instrumentation*

After inducing anaesthesia, animals were tracheotomized and connected to mechanical ventilation (Servo-i. Maquet Critical Care, Solna, Sweden). Intravenous anaesthesia was then maintained using a combination of ketamine, midazolam and fentanyl, adding rocuronium for muscle relaxation after adequate anaesthesia was ascertained. Baseline ventilation settings consisted of positive end expiratory pressure (PEEP) 8cmH2O, tidal volume 6ml/kg, inspiratory/expiratory ratio (I:E) 1:2, FIO$_2$ 1 and respiratory rate adjusted to keep an end-tidal CO$_2$ around 45mmHg in volume-controlled ventilation. A 4-lumen central catheter and a PA catheter (Edwards, Irving, CA, USA) were inserted through the right internal jugular vein. A small left lateral thoracotomy was performed to expose the PA trunk around which an ultrasonic flow probe (Confidence Flowprobes, PAU series, Transonic, Ithaca, NY, USA) was placed as proximal to the RV as possible. A micro-tip pressure transducer (Mikro-Tip, SPR-340, Millar, Houston, TX, USA) was inserted directly into the PA at the level of the ultrasonic probe. Thereafter, the thorax was carefully closed layer by layer.

Monitoring also included electrocardiogram; femoral artery pressure for arterial gas samples and transpulmonary thermodilution through a PiCCO (Pulsion, Germany); and airway flow and pressure measured through a pneumotachograph attached to the endotracheal tube and a NICO (Philips, Wallingford, CT, USA).

After instrumentation, animals were subjected to a lung volume history homogenization manoeuvre by changing to pressure-controlled ventilation mode with PEEP 20cmH2O, inspiratory driving pressure 20cmH$_2$O, respiratory rate 20 bpm and I:E 1:1 for 30 seconds. Baseline measurements were obtained 15 min thereafter.

*ARDS model*

Saline (30ml/kg) lung lavages were performed until a PaO$_2$/FIO$_2$ < 200mmHg was reached at PEEP 8cmH$_2$O and FIO$_2$ 1.0. Animals were then subjected to two hours of injurious ventilation (PEEP 0cmH$_2$O and inspiratory driving pressure 35cmH$_2$O). Once the model was established, ventilation parameters were changed to baseline and a new set of measurements was obtained after stabilization for one hour. Intravenous boluses of normal saline (5ml/kg) were administered if mean systemic arterial pressure was < 60mmHg during the model creation. Two to three continuous minute stable signal period was selected at baseline and ARDS avoiding extrasystoles and other undesired variability sources.

*Signal acquisition and processing*

The flow probe was attached to a Perivascular Flow Module (TS420, Transonic, Ithaca, NY, USA) and the pressure probe to a PowerLab Data Acquisition System (PowerLab 16/35, Adinstruments, Dunedin, New Zealand). Both systems were connected to a MP-150 Biopac Data Acquisition System (MP-150WSW, Biopac Systems, Inc., Goleta, CA, USA) for simultaneous data acquisition together with ECG, airway pressure and flow and other physiological signals at 200 or 1000 hz (then resampled to 200 hz). Signals were processed using LabChart 8 software (Adinstruments, Dunedin, New Zealand). For continuous analysis, signal were smoothed applying a Savitzky-Golay 2nd order filter with an 11 points window. All signal analysis was performed using customized routines programmed in Excel (Microsoft Corporation, Redmond, WA, USA) and Matlab (Mathworks, Inc, Natick MA, USA).

*Correction of the ventilation effect on continuous measurement of PA pressure*

Airway pressure changes during ventilation and is transmitted to the pleural space and this affects PA pressure measurement. In this study we applied a method for correcting this effect by tracking the PA pressure diastolic change along the respiratory cycle. From a physiologic point of view, the transmission of an external pressure to a vessel should be higher in late diastole as most of the pressure resulting from cardiac contraction has gone at this point. Based on this, to track the effect airway pressure on PA, the minimum pressure at the start of each PA pressure cycle was determined. Then, cardiac beats were reordered according to the time at which this minimum pressure occurs during the respiratory cycle. A signal consisting on the value of this minimum points along the respiratory time was then created. A smoothing splines algorithm was applied to estimate missing signal at some time points by interpolation allowing slight signal smoothing but preserving main curve shape. For this, the curve was repeated avoiding distortion at start and end assuming continuity and cyclic invariability during the evaluated period. Then, signal was scaled to its minimum value and this new function was applied to all the evaluated time period at intervals defined by respiratory cycle assuming such invariability. Finally, this signal was subtracted from measured PA pressure. An example of the correction of PA pressure in shown in Figs. 7A-C.

Beat by beat Measurement of Stroke Volume and PWA Variables to Estimate It

Flow and PA pressure (corrected signal) foots were detected and an algorithm starting at each new pressure and flow cycle was applied to measure at each beat:

- Stroke volume reference (SVref): Area under the flow-time curve during systole.
- PA pulse pressure (PP): Systolic minus foot PA pressure.
- Systolic PA pressure area (SystAUC): Area under the PA pressure curve starting at pressure foot and ending at dicrotic notch.
- Standard deviation (SD) of PA pressure waveform: SD of pressure along each pressure cycle.

The performance of PA PP, SD and SystAUC measured from the non-corrected and corrected pressure signals as variables to estimate SV was evaluated.

*Evaluation of the effect of breathing on estimation of RV SV based on PA PWA*

Pulse pressure, standard deviation of the pressure cycle, and systolic pressure area have demonstrated to be useful for the estimation of SV when calculated from the systemic pressure waveform. For such an application a calibration factor is used. There have been described several ways to obtain this factor and a detailed explanation of these is beyond the scope of this study. This factor depends mainly on vascular properties and it is assumed to be constant along the respiratory cycle. To demonstrate the application of the new method for the estimation of SV a calibration factor was obtained by an independent measurement of cardiac output by transpulmonary thermodilution (repeated by three at each evaluated condition). This cardiac output was scaled to the mean PA flow measured by the transonic system in all the experiments. Then the calibration factor was obtained as the quotient between the scaled thermodilution cardiac output divided by heart rate and the evaluated variable (non-corrected and corrected PA PP, SD and SystAUC). The ability of PA PWA variables to beat by beat track SV along stable conditions (baseline and after ARDS) in which the most of SV variability depends on respiration was also evaluated without applying the calibration factor.

As beat by beat SVref and PA PWA variables did not follow a normal distribution during period of analysis the median value during this period was chosen as representative. For the same reason the median absolute deviation (MAD) and the MAD divided by median (MAD/MED) were chosen to evaluate variability of evaluated variables.

A very well described parameter was used to evaluate the effect of breathing on SV, the SV variation (SVV):

$$SVV = \frac{SV_{max} - SV_{min}}{SV_{mean}}$$

where SVmax and SV min are the maximum and minimum SV during a respiratory cycle and the SVmean may be computed as:

$$SV_{mean} = \frac{SV_{max} + SV_{min}}{2}$$

We estimated the SVV using each of the PA PWA variables and from the SVref. For this, SVV was calculated during each respiratory cycle and the mean value of all of these measurements during the studied period was chosen as representative.

Frequency domain analysis was applied to evaluate the effect of ventilation correction on respiratory and cardiac components of PA pressure. For this analysis, amplitude spectrum was constructed on PA pressures segments of approximately 2 minutes applying a Fast Fourier Transform algorithm with a 16384 size, a Hamming window and 75% overlapping. Noise was defined as a value less than 1% of the maximum amplitude of the corresponding spectrum. Respiratory and cardiac components were defined according to the measured respiratory and heart rated during each period of study. The sum of area under the curve of the 1-4 harmonics was used for calculating the respiratory component and the sum of 1-12 harmonics for the cardiac component.

The flow diagram of the method applied to estimate SV and its variability based on a correction of the effect of ventilation on PA pressure is summarized in Fig. 6.

# EP 4 417 117 A1

*Statistical analysis*

Normality was assessed using the Shapiro-Wilk test. Data were expressed as mean (standard deviation) if followed a normal distribution or median [interquartile range] if not. Paired t-test and Wilcoxon signed-rank test (for non-normally distributed data) were used for comparing measurements in Baseline versus ARDS and for comparing corrected versus non-corrected PA pressure derived variables. Two-way repeated measures ANOVA was applied to evaluate the effect of ventilation correction and lung condition (Baseline and ARDS), on PA PWA derived variables. Pearson's R (for normal distributed data) or Spearman's Rho (for non-normal distributed data) were calculated to evaluate the correlation between SVref and PA PWA variables. Cross correlation between SVref and PA PWA variables was evaluated to test if the transmission of pressure to PA or the applied correction caused a phase shift between SV estimated from the flow (SVref) and pressure signal. A phase shift was defined as an increased in correlation in any lag between $\pm 1$ to $\pm 4$. Significance was considered at $p < 0.05$. Statistical analysis was performed with Microsoft excel 2013 (Microsoft Corporation, Redmond, WA, USA) and Stata v15 (StataCorp, College Station, TX, USA).

## Results

Respiratory mechanics and function and pulmonary hemodynamics variables are presented in table 1.

**Table 1: Respiratory and pulmonary hemodynamic variables during the studied situations.**

| Variable | Baseline | ARDS | p |
|---|---|---|---|
| $V_T$ (ml/kg) | 8.48 [7.04-8.59] | 8.52 [5.85-8.76] | 0.500 |
| RR (bpm) | 32 (2) | 33 (3) | **0.570** |
| PEEP ($cmH_2O$) | 8.1 (0.1) | 8 .1 (0.2) | 0.711 |
| PPlat $cmH_2O$) | 15.5 [12.9-15.7] | 27.7 [26.9-29.9] | **0.043** |
| MAwP ($cmH_2O$) | 10.9 (0.6) | 14.4 (0.7) | **<0.001** |
| DP ($cmH_2O$) | 7.3 [4.9-7.5] | 19.4 [18.8-22.2] | **0.043** |
| Cdyn (ml/$cmH_2O$) | 39.5 (7.4) | 11.8 (2.3) | **0.002** |
| SI | 1.04 [0.95-1.06] | 1.16 [1.16-1.16] | **0.043** |
| $PaO_2$ (mmHg) | 541 (49) | 196 (108) | **0.005** |
| $PaCO_2$ (mmHg) | 42 (6) | 59 (12) | **0.019** |
| $SaO_2$ (%) | 98.8 (0.8) | 95.8 (2.82) | 0.069 |
| pH | 7.43 (0.06) | 7.27 (0.06) | **0.027** |
| Shunt | 0.09 (0.05) | 0.25 (0.10) | 0.060 |
| CO (l/min) | 3.19 [2.91-3.2] | 2.71 [2.38-3.82] | 0.500 |
| HR (bpm) | 96 (17) | 107 (21) | 0.257 |
| MPAP (mmHg) | 20 (2) | 31 (6) | 0.007 |
| PP (mmHg) | 11 (4) | 19 (2) | 0.009 |
| PVR (dyn.s.cm$^{-5}$) | 172 (65) | 577 (156) | 0.005 |
| PComp (ml/mmHg) | 3.37 [3.10-5-65] | 1.71 [1.35-2.04] | 0.043 |
| EAeff (mmHg/ml) | 0.62 (0.10) | 1.11 (0.26) | 0.008 |

The abbreviations of table 1 are now described: $V_t$ tidal volume, RR respiratory rate, $SaO_2$ arterial haemoglobin $O_2$ saturation, PEEP positive end expiratory pressure, PPlat plateau pressure, MAwP mean airway pressure, DP driving pressure (DP=PPlat-PEEP), Cdyn respiratory system dynamic compliance, SI stress index, CO cardiac output, HR heart rate, MPAP mean pulmonary artery pressure, PP pulmonary artery pressure, PVR pulmonary vascular resistance, PComp pulmonary artery compliance (stroke volume divided by pulmonary artery pulse pressure). EAeff effective arterial elastance (mean minus wedge pulmonary artery pressure divided by stroke volume).

As expected, ARDS resulted in significant worsening of respiratory mechanics and gas exchange and on pulmonary hemodynamics. ARDS leaded to cyclic overdystension as demonstrated by an increase in stress index to values above 1.1.

The PA PWA variables are shown in table 2a.

**Table 2a Evaluation of the effect of correction on variables to estimate stroke volume during the studied conditions.**

| Variable Correction | Cond | SVref | PP NC | PP Cor | SD NC | SD Cor | SystAUC NC | SystAUC Cor |
|---|---|---|---|---|---|---|---|---|
| Median | BL | 35.8 (2.9) ml | 11.5 (4.6) mmHg | 11.4 (4.4) mmHg | 3.67 (1.51) mmHg | 3.79 (1.59) mmHg | 7.78 (1.26) mmHg/s | 7.52 (1.26) mmHg/s |
| | ARDS | 31.2 (7.1) ml | 18.0 (2.0) mmHg | 17.8 (2.2) mmHg[a,c] | 5.28 (0.76) mmHg | 5.58 (0.90) mmHg[a,c] | 9.58 (1.99) mmHg/s | 9.19 (1.91) mmHg/s[a,c] |
| MAD | BL | 2.2 (0.6) ml | 1.1 (0.3) mmHg | 0.5 (0.2) mmHg | 0.15 (0.03) mmHg | 0.20 (0.11) mmHg | 0.38 (0.14) mmHg/s | 0.24 (0.11) mmHg/s |
| | ARDS | 2.1 (0.4) ml | 1.7 (0.4) mmHg | 0.8 (0.1) mmHg[a,c] | 0.23 (0.01) mmHg | 0.28 (0.08) mmHg[a] | 0.50 (0.18) mmHg/s | 0.38 (0.15) mmHg/s[a,c] |
| MAD/med | BL | 0.06 (0.02) | 0.11 (0.03) | 0.05 (0.01) | 0.05 (0.01) | 0.05 (0.01) | 0.05 (0.02) | 0.03 (0.01) |
| | ARDS | 0.07 (0.02) | 0.09 (0.01) | 0.05 (0.00)[c] | 0.04 (0.01) | 0.05 (0.01) | 0.05 (0.01) | 0.04 (0.01)[c] |
| Phase shift (fraction)* | BL | | 0.2 | 0.2 | 0.8 | 0.2 | 1 | 0 |
| | ARDS | | 0.6 | 0 | 1 | 0[c] | 1 | 0[c] |
| Rho SV | BL | | 0.863 (0.70) | 0.882 (0.092) | -0.111 (0.342) | 0.876 (0.130) | 0.543 (0.141) | 0.923 (0.050) |
| | ARDS | | 0.752 (0.080) | 0.848 (0.049) | -0.033 (0.262) | 0.839 (0.077)[c] | 0.483 (0.114) | 0.928 (0.026)[c] |
| SVV (%) | BL | 20 (6) | 31 (6) | 18 (4) | 17 (9) | 20 (6) | 14 (4) | 12 (6) |
| | ARDS | 23 (5) | 37 (7) | 23 (6)[c] | 17 (3) | 25 (7) | 15 (3) | 17 (6)[a,c] |

Median, MAD (median absolute difference) and MAD/MED (MAD divided by median) are obtained from all the beat-by-beat measurements during the studied period. Phase shift show the fraction of the studied pigs in which correlation between SVref and SV estimated from PA PWA improves in the cross-correlation analysis in any lag between ±1 and ±4. Rho SV is the correlation between SVref and the corresponding variable. SVV is the stroke volume variation.

The representative value of each variable is the mean or median value (according to distribution of data) for the 5 animals in the evaluated condition.

- $^c$p < 0.05 for the correction
- $^s$p < 0.05 for lung condition
- $^i$p < 0.05 for the interaction correction lung condition

Correction changed the PP, SD and SystAUC. Interestingly, this change was not equal for all variables as correction decreased PP and SystAUC while increased the SD. Correction also decreased the variability of PP and SystAUC as reflected by the MAD and MAD/MED.

The RV SV obtained from the PA PWA variables is shown in table 2b.

**Table 2b. Evaluation of the effect of correction on variables to estimate stroke volume during the studied conditions.**

| Variable | Cond | SVref | PP SV | | SD SV | | SystAUC SV | |
|---|---|---|---|---|---|---|---|---|
| Correction | | | NC | Cor | NC | Cor | NC | Cor |
| Median | BL | 35.8 (2.9) | 31.0 (3.7) | 31.0 (4.0) | 29.6 (3.6) | 31.1 (3.9) | 29.7 (3.5) | 30.8 (3.8) |
| (ml) | ARDS | 31.2 (7.1) | 35.0 (9.8) | 34.6 (9.4)c | 32.5 (9.3) | 34.7 (9.5)c | 33.1 (9.4) | 34.1 (9.3)c |
| MAD | BL | 2.2 (0.6) | 3.4 (1.1) | 1.5 (0.3) | 1.4 (0.4) | 1.5 (0.3) | 1.4 (0.5) | 1.0 (0.4) |
| (ml) | ARDS | 2.1 (0.4) ml | 3.2 (0.6) | 1.6 (0.5)c | 1.4 (0.7) | 1.8 (0.7) | 1.7 (0.5) | 1.3 (0.5)c |
| MAD/med | BL | 0.06 (0.02) | 0.11 (0.03) | 0.05 (0.01) | 0.05 (0.01) | 0.05 (0.01) | 0.05 (0.02) | 0.03 (0.01) |
| | ARDS | 0.07 (0.02) | 0.09 (0.01) | 0.05 (0.00)c | 0.04 (0.01) | 0.05 (0.01) | 0.05 (0.01) | 0.04 (0.01)c |
| Phase shift | BL | | 0.2 | 0.2 | 0.8 | 0.2 | 1 | 0 |
| (fraction)* | ARDS | | 0.6 | 0 | 1 | 0c | 1 | 0c |
| Rho SV | BL | | 0.863 (0.70) | 0.882 (0.092) | -0.111 (0.342) | 0.876 (0.130) | 0.543 (0.141) | 0.923 (0.050) |
| | ARDS | | 0.752 (0.080) | 0.848 (0.049) | -0.033 (0.262) | 0.839 (0.077)c | 0.483 (0.114) | 0.928 (0.026)c |
| SVV (%) | BL | 20 (6) | 31 (6) | 18 (4) | 17 (9) | 20 (6) | 14 (4) | 12 (6) |
| | ARDS | 23 (5) | 37 (7) | 23 (6)c | 17 (3) | 25 (7) | 15 (3) | 17 (6)i,c |

This table is mainly showed as prove of the application of the proposed method and algorithm. The resulting median values are mainly affected by the calibration factor; however the effect of corrections remains in the same direction as in table 2a. This also applies for the MAD. As expected, the results of the rest of variables in table 2b are equal than in table as they depend on the beat-by-beat variability and not on the absolute value of SV.

Fig. 4 shows a simultaneous recording of PA pressure (Non-Corrected and Corrected) and flow (Figs, 4A, 4C) and the beat-by-beat estimation of SystAUC and RV SVref (Figs, 4B, 4D). A decoupling between SystAUC and SVref is observed for the Non-Corrected signal and not on the Corrected signal. This is observed also in the scatter plot of SVref vs SV calculated from SystAUC (Fig. 1A). This effect was partially corrected by shifting the signals (Fig. 1B) and completely removed by correcting the effect of ventilation by the proposed method (Fig. 1C). This effect was evaluated by cross correlation finding a phase shift in SD and SystAUC that was corrected by correction (table 2a and 2b). A similar trend was observed for PP in which signals were shifted in 60% if the cases in baseline for Non-Corrected pressure although correction did not find significance. Finally, a huge increase in the beat-by-beat correlation between SVref and SD and between SVref and SystAUC resulted from the correction of PA pressure. A trend to increase the correlation between SVref and PP with the correction was also observed but no reached statistical significance.

An example of the effect of correction on PA pressure amplitude spectrum is shown in Figs. 2A-B. Flow amplitude spectrum is shown in upper panel for reference. Correction decreased the respiratory component without affecting the cardiac component. This effect occurred in baseline and also during ARDS (Fig. 3).

Correction decreased the SVV calculated from PP and increased the SVV calculated from SystAUC (table 2a and 2b). A trend to increase the SVV calculated form SD was observed after correction. Overall, the correlation between SVVref and SVV calculated from corrected PA pressure was better than between SVVref and SVV calculated from non-corrected PA pressure (Fig. 10 and table 3).

### Table 3: correlation between SVVref and SVV

| Correlation with SVV ref | R2 | p |
| --- | --- | --- |
| **Baseline** | | |
| SVV PP NC | 0.0293 | 0.783 |
| SVV SD NC | 0.685 | 0.084 |
| SVV SystAUC NC | 0.279 | 0.361 |
| SVV PP Cor | 0.977 | 0.002 |
| SVV SD Cor | 0.745 | 0.060 |
| SVV SystAUC Cor | 0.756 | 0.060 |
| **ARDS** | | |
| SVV PP NC | 0.918 | 0.010 |
| SVV SD NC | 0.299 | 0.340 |
| SVV SystAUC NC | 0.839 | 0.785 |
| SVV PP Cor | 0.988 | 0.001 |
| SVV SD Cor | 0.861 | 0.023 |
| SVV SystAUC Cor | 0.858 | 0.024 |

SVVref, SVV PP, SVV SD and SVV SystAUC stroke volume variation obtained from transonic flow measurement, pulse pressure, standard deviation of pressure and systolic pressure area of pulmonary artery respectively. NC and Cor stands for measurements obtained from the Non-Corrected and Corrected PA pressure respectively

In these experimental conditions a clear improvement in the RV SV estimation from PA PWA resulted after correcting the effect of ventilation on continuous PA pressure measurement. To the best of our knowledge, this is the first time that this approach has been implemented. These results can be relevant for clinical practice as better algorithms to monitor RV SV, RV function and pulmonary hemodynamics can be developed leading to better patient outcomes.

The improvement in the RV SV estimation was demonstrated for the three evaluated variables: PP, SD and SystAUC. An evidence of this is the better correlation between SVref and the PA PWA variables when the correction was applied. A possible partial phase shift could explain the low correlation between beat-by-beat SWref and the estimated PA PWA variables from Non-Corrected PA pressure. This could be attributed to a modification of the measured pressure in certain stages of the PA pressure cycle leading to a "wrong" calculus of the PA PWA variable. For example, the RV SV at the beginning of inhalation in a fully mechanically ventilated subject should be higher than at the beginning of exhalation as for the first preceding diastole occurs during a period of low intrathoracic pressure leading to a higher RV filling. However, the farthest the PA pressure cycle start during inhalation a mayor pressure will be transmitted increasing the pressure points value at early systole leading to a possible lowest PA pressure amplitude and systolic area. In other words, the measured beat by beat measured PA pressure depends not only on the effect cardiac contraction and physiologic heart lung interactions but also on the transmission of airway pressure. If the airway pressure during ventilation does not follow the expected physiologic changes in RV function and load a decoupling between the PA PWA estimated SV and the real SV will occur. This was supported by the partial improve in correlation in the phase shift analysis (table 2a and 2b) and illustrated in Figs. 1 and 4.

Another relevant finding is that the correction kept intact the cardiac component estimated from the PA pressure amplitude spectrum analysis (Figs. 2 and 3). This could be related with preservation of the contribution of the heart to the PA pressure after the correction. The effect of the heart on pressure depends mainly on the ventricle load and function, the vascular properties and the interactions between them, and the resulting SV is also the determined by these factors. In the other hand, the respiratory component decreased after correction which means that the effect of ventilation on the corrected PA pressure signal decreased. This can be interpreted as the result of the elimination of the effect of transmitted pressure while keeping the physiologic effect of ventilation on heart load. This physiologic effect leads to a modulation on SV. In this regard, the combined effect of correction on cardiac and respiratory components could lead to a better estimation of the effect of ventilation on SV. This can be evaluated by the estimation of SVV. As demonstrated in Fig. 10 and table 3, the correlation between SVV ref and SVV obtained from PA PWA variables improved after correction. This supports the concept that correction leads to a better estimation not only of SV but also of its modulation by ventilation. Due to clinical relevance of this, a flow diagram of the application the actual method in clinical practice with the purpose of assessing SVV is shown in Fig. 9.

**Claims**

1. A computer-implemented method for estimating right ventricular stroke volume from the pulmonary artery pressure, comprising:

    receiving data indicative of two or more corrected pulmonary artery pressure measurements, wherein the two or more corrected pulmonary artery pressure measurements are one or more pulmonary artery pressure measurements over one or more cardiac cycles at one or more points in the respiratory cycle, each point corrected for the external airway pressure exerted on the heart during the cardiac cycle at the respective point in the respiratory cycle, the correction comprising a subtraction of a respiratory correction function for all of the pulmonary artery pressure measurements; and
    estimating the right ventricular stroke volume for each of the one or more cardiac cycles based on a parameter of the one or more corrected pulmonary artery pressure measurements using a predetermined relationship between the parameter and right ventricular stroke volume.

2. The computer-implemented method according to claim 1, wherein the respiratory correction function is computed through a method comprising the steps:

    a. receiving data indicative of two or more respiratory cycles;
    b. receiving data indicative of two or more pulmonary artery pressure cycles, wherein the pulmonary artery pressure data is synchronized with the respiratory data;
    c. detecting a predetermined position of the at least two, preferably three, more preferably four, more preferably five, more preferably 6, 7, 8, 9, 10, 11, 12, 13, 14, 15, 16, 17, 18, 19 or at least preferably 20, consecutive or non-consecutive, respiratory cycles of step a), and rearranging each pulmonary artery pressure cycle of step b) according to their position in time, with respect to the aforesaid predetermined position of these respiratory cycles;
    d. defining a discrete signal by plotting each of a determined measurement of the pulmonary artery pressure cycles in relation to the time of its respective respiratory cycle's starting point obtained as described in step c) above, as an ordinate within the respiratory cycle; and

e. computing the respiratory correction function by reconstructing a continuous signal from the defined discrete signal.

3. The computer-implemented method according to claim 2, wherein the reconstruction of the continuous signal from the discrete signal is done through interpolation, preferably wherein the interpolated signal is performed through a smoothing splines algorithm.

4. The computer-implemented method according to any of the claims 2 to 3, wherein the predetermined position of the respiratory cycle is the starting position of the respiratory cycle.

5. The computer-implemented method according to any of the claims 1 to 4, wherein the correction comprises subtracting to each of the pulmonary artery pressure measurements the value of each corresponding measurement of the respiratory correction function, wherein the respiratory correction function is synchronised in time with the pulmonary artery pressure measurements according to their respective point in the respiratory cycle, and wherein the respiratory correction function is extended in a repeated pattern at intervals defined by respiratory cycle.

6. The computer-implemented method according to any of the claims 2 to 5 when dependent on claim 2, wherein the determined measurement of the pulmonary artery pressure cycle is the final diastolic pressure value of each pulmonary artery pressure cycle.

7. The computer-implemented method according to any of the claims 1 to 6, wherein the parameter is the difference between the systolic and diastolic corrected pressure in one cardiac cycle.

8. The computer-implemented method according to any of the claims 1 to 6, wherein the parameter is the area under the curve of the systolic portion of the corrected pressure in one cardiac cycle.

9. The computer-implemented method according to any of the claims 1 to 6, wherein the parameter is the standard deviation of the corrected pressure over one cardiac cycle.

10. The computer-implemented method according to any of the claims 1 to 9, wherein the respiratory correction function is scaled such that its minimum value is 0.

11. The computer-implemented method according to any of the claims 1 to 10, wherein the predetermined relationship is a linear relationship computed through a calibration function between the right ventricular stroke volume and the parameter of the pulmonary artery pressure, preferably wherein the calibration function is the quotient between the scaled thermodilution cardiac output divided by heart rate and the parameter of the of the pulmonary artery pressure.

12. A computer-implemented method for estimating stroke volume variation (SVV) over the respiratory cycle from the pulmonary artery pressure, comprising:

a) estimating right ventricular stroke volume (SV) according to any of the methods of claims 1 to 11,
b) estimating SVV over the respiratory cycle for each respiratory cycle as:

$$SVV = \frac{SV_{max} - SV_{min}}{SV_{mean}}$$

wherein SVmax is the maximum SV during such cycle, SVmin is the minimum SV during such cycle, and SVmean is the mean SV during such cycle.

13. An apparatus comprising a processor and a memory unit, wherein the memory comprises instructions that when executed by the processor, estimates the right ventricular stroke volume according to any of the computer-implemented methods of claims 1 to 11.

14. The apparatus according to claim 13, wherein the memory unit further comprises instructions that, when executed by the processor, estimates stroke volume variation (SVV) over the respiratory cycle from the pulmonary artery pressure according to the computer-implemented method of claim 12.

15. A computer program product configured for estimating a right ventricular stroke volume from a ventricle arterial pressure according to any of the computer-implemented methods of claims 1 to 11, preferably wherein the computer program product is further configured to estimate stroke volume variation (SVV) over the respiratory cycle from the pulmonary artery pressure according to the computer-implemented method of claim 12.

Fig. 1A

Fig. 1B

Fig. 1C

PA Pressure (Non-Corrected) Spectrum

PA Pressure (Corrected) Spectrum

Fig. 2A

Fig. 2B

Fig. 3A

Fig. 3B

Fig. 4C

Fig. 4A

Fig. 4D

Fig. 4B

Fig. 5A

Fig. 5B

Fig. 5C

Fig. 6

Fig. 7A

Fig. 7B

Fig. 7C

| | r2 pp-vs | r2 auc-vs | r2 sd-vs | r2 pp-vs corr | r2 auc-vs corr | r2 sd-vs corr |
|---|---|---|---|---|---|---|
| 1203bl | 0.933 | 0.130 | 0.017 | 0.934 | 0.812 | 0.867 |
| 1203ards | 0.650 | 0.175 | 0.003 | 0.781 | 0.854 | 0.755 |
| 3005bl | 0.583 | 0.028 | 0.203 | 0.481 | 0.832 | 0.369 |
| 3005ards | 0.744 | 0.133 | 0.013 | 0.787 | 0.910 | 0.766 |
| 1609bl | 0.854 | 0.343 | 0.039 | 0.913 | 0.956 | 0.972 |
| 1609ards | 0.747 | 0.264 | 0.089 | 0.949 | 0.972 | 0.950 |
| 1809bl | 0.870 | 0.139 | 0.016 | 0.922 | 0.296 | 0.956 |
| 1809ards | 0.848 | 0.354 | 0.022 | 0.872 | 0.902 | 0.933 |
| 1909bl | 0.928 | 0.421 | 0.397 | 0.976 | 0.992 | 0.986 |
| 1909ards | 0.807 | 0.545 | 0.107 | 0.920 | 0.952 | 0.900 |
| 0811bl | 0.833 | 0.518 | 0.014 | 0.898 | 0.921 | 0.929 |
| 0811ards | 0.676 | 0.327 | 0.323 | 0.391 | 0.683 | 0.557 |
| Mean | 0.789 | 0.281 | 0.104 | 0.819 | 0.840 | 0.828 |
| T test corr vs non corr | | | | 0.426 | 0.000 | 0.000 |

Fig. 8

Fig. 9

Fig. 10A

Fig. 10B

Fig. 10C

Fig. 10D

Fig. 10E

Fig. 10F

Fig. 11

Fig. 12

| Europäisches Patentamt European Patent Office Office européen des brevets | **EUROPEAN SEARCH REPORT** | Application Number EP 23 38 2143 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/158604 A1 (CONSORCIO CENTRO DE INVESTIG BIOMEDICA EN RED M P [ES] ET AL.) 22 August 2019 (2019-08-22) * the whole document * | 1-15 | INV. A61B5/029 A61B5/00 A61B5/021 A61B5/08 A61M16/00 |
| A | DAVID BERGER ET AL: "Right ventricular stroke volume assessed by pulmonary artery pulse contour analysis", INTENSIVE CARE MEDICINE EXPERIMENTAL, BIOMED CENTRAL LTD, LONDON, UK, vol. 8, no. 1, 7 October 2020 (2020-10-07), pages 1-13, XP021282641, DOI: 10.1186/S40635-020-00347-7 * Items "Introduction" and "Tested methods" on pages 1-3- * | 1-15 | |

TECHNICAL FIELDS SEARCHED (IPC)

A61B

The present search report has been drawn up for all claims

| Place of search | Date of completion of the search | Examiner |
|---|---|---|
| The Hague | 19 July 2023 | Dhervé, Gwenaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 38 2143**

This annex lists the patent family members relating to the patent documents cited in the above-mentioned European search report.
The members are as contained in the European Patent Office EDP file on
The European Patent Office is in no way liable for these particulars which are merely given for the purpose of information.

**19-07-2023**

| Patent document cited in search report | | Publication date | Patent family member(s) | | Publication date |
|---|---|---|---|---|---|
| WO 2019158604 | A1 | 22-08-2019 | EP | 3752047 A1 | 23-12-2020 |
| | | | WO | 2019158604 A1 | 22-08-2019 |

EPO FORM P0459

For more details about this annex : see Official Journal of the European Patent Office, No. 12/82